# EUROPEAN PATENT APPLICATION

(11) **EP 4 059 552 A1**
(43) Date of publication of application: **21.09.2022**
(21) Application number: 21163543.8
(22) Date of filing: 18.03.2021
(51) Int. Cl.: A61M 11/04, A24F 40/00, A24F 40/10, A24F 40/50, A61M 15/06, H02J 7/00

(54) **CONTROL DEVICE FOR CONTROLLING ELECTRICAL POWER SUPPLY IN AN AEROSOL GENERATION DEVICE**

(71) Applicant: JT International SA, 1202 Geneva (CH)
(72) Inventor: COBURN, Broderick, 1185 Mont-sur-Rolle (CH); SECO, Joao, 1202 Geneva (CH)
(74) Representative: Serjeants LLP

(57) **Abstract**

A control device (1) equips an aerosol generation device (2) comprising a porous body (3) storing liquid substance until vaporized and a heater (4) arranged, when supplied with electrical power, for heating this porous body (3) to vaporize the liquid substance for generating an aerosol. This control device (1) comprises at least a processor (14) and a memory (15) arranged for performing operations consisting of controlling supply of the heater (4), during a vaping session comprising successive inhalation phases, with a maximal electrical power that decreases between the first and last inhalation phases.

## Description

### Field of the invention

The present invention relates to an aerosol generation device, and more precisely to the control of the supply of electrical power in such a device.

### Background

Some aerosol generation devices comprise at least:
- a battery (or power source) storing electrical energy and possibly rechargeable,
- a porous body storing liquid substance until vaporized,
- a heater arranged, when supplied with electrical power, for heating this porous body to vaporize this liquid substance for generating an aerosol (or mist), and
- a control device (or controller) electrically coupled to the battery and controlling supply of this heater with electrical power during a vaping session comprising successive inhalation phases during which a user inhales the generated aerosol.

The aerosol is released into a flow path extending between an inlet and an outlet of the aerosol generation device. This outlet may be arranged as a mouthpiece through which the user inhales the generated aerosol (or mist), but this is not mandatory.

The aerosol generation device may be portable, i.e. usable when held by a user. In this case it may be a vaporizer or an electronic cigarette.

In the following description the term "aerosol" may include a suspension of substance as one or more of solid particles, liquid droplets and gas. Such a suspension may be in a gas including air. Aerosol herein may generally refer to, or include, a vapor, and may include one or more components of the substance.

The liquid substance may comprise one or more of nicotine, polyol, caffeine or other active components. An active component may be carried by a liquid carrier which may include propylene glycol or glycerin, for instance. A flavoring may also be present in the liquid substance and may include Ethylvanillin (vanilla), menthol, Isoamyl acetate (banana oil) or similar, for instance.

To avoid a permanent generation of aerosol during a vaping session, the heater is only supplied with an electrical power adapted to the vaporization after a pre-heating phase and once the beginning of an inhalation phase (or "puff") has been detected (i.e. each time the user starts to inhale).

As described in the patent document US 2018/0289909, the porous body may be a tablet, cylinder or disk, and may be a piece of porous ceramic, sintered glass, alumina or sintered metal soaked, injected or infused with liquid in order that the liquid substance is held in place by capillary action (and also possibly stored in a cavity).

The quantity of liquid substance stored in the porous body is limited, and depends on the porosity and size of this porous body. Typically, the number of inhalation phases (or puffs) that a user can have during a vaping session is smaller than twenty.

During a vaping session, the quantity of liquid substance stored in the porous body decreases after each inhalation phase, and therefore the heat capacity of the porous body decreases. So, as the electrical power supplying the heater is approximately constant during all the inhalation phases, the temperature of the porous body keeps increasing during a vaping session, which may induce vaporization between two inhalation phases and therefore a reduction of the number of puffs per porous body and/or quality inhomogeneities between puffs, and/or an aerosol temperature greater than the expected one during an inhalation phase, which may cause a discomfort to the user. Due to the increasing porous body temperature it may also cause an increase and inconsistency in vapor output between successive puffs and/or alter the taste or cause taste inconsistency and/or cause inconsistency of the constituents and produce higher levels of undesirable constituents typically produced at higher temperatures.

So, the present invention aims at improving the situation.

### Summary

The proposed invention provides an embodiment of a control device:
- intended for equipping an aerosol generation device comprising a porous body storing liquid substance until vaporized and a heater arranged, when supplied with electrical power, for heating this porous body to vaporize this liquid substance for generating an aerosol, and
- comprising at least a processor and a memory arranged for performing operations consisting of controlling supply of this heater with electrical power during a vaping session comprising successive inhalation phases during which a user inhales the generated aerosol.

This control device is characterized in that its processor and memory are arranged for performing operations consisting of controlling supply of the heater, during the vaping session, with a maximal electrical power that decreases between a first inhalation phase and a last inhalation phase.

Thanks to this control of the supplied electrical power the consequence of the decrease of the porous body heat capacity is at least partly compensated, and therefore the temperature of the porous body does not increase significantly from an inhalation phase to the following one, which prevents vaporization between two inhalation phases or perceptible temperature variations and compositions of the aerosol.

The embodiment of control device may comprise other features, considered separately or combined, and notably :
- its processor and memory may be arranged for performing operations consisting of controlling supply of the heater with an approximately constant maximal electrical power and then an approximately constant first intermediate electrical power, smaller than this approximately constant maximal electrical power, during respectively first and second successive parts of each inhalation phase, the approximately constant maximal electrical power decreasing between the first and last inhalation phases;
- its processor and memory may be arranged for performing operations consisting of controlling supply of the heater with an approximately constant first intermediate electrical power that decreases between the first and last inhalation phases;
- its processor and memory may be arranged for performing operations consisting of controlling supply of the heater with an approximately constant second intermediate electrical power after an end of an inhalation phase and before a beginning of a following inhalation phase;
- its processor and memory may be arranged for performing operations consisting of controlling supply of the heater, before an inhalation phase, with an approximately constant second intermediate electrical power that is lower than the approximately constant first intermediate electrical power of this inhalation phase;
- its processor and memory may be arranged for performing operations consisting of controlling supply of the heater with an approximately constant pre-heating electrical power during a pre-heating phase occurring before the first inhalation phase;
- its processor and memory may be arranged for performing operations consisting of controlling supply of the heater with an approximately constant pre-heating electrical power that is greater than the maximal electrical power supplied during the first inhalation phase;
- its processor and memory may be arranged for performing operations consisting of controlling an end of electrical power supply of the heater when occurs an event chosen from a group comprising a termination of the vaping session by the user, an elapsed time having reached a first limit since a last inhalation phase, and a duration of the vaping session exceeding a second limit.

The proposed invention also provides an embodiment of an aerosol generation device comprising:
- a battery storing electrical energy,
- a porous body storing liquid substance until vaporized,
- a heater arranged, when supplied with electrical power, for heating this porous body to vaporize this liquid substance for generating an aerosol during a vaping session comprising inhalation phases during which a user inhales this generated aerosol, and
- the control device presented above and electrically coupled to this battery.

For instance, this aerosol generation device may constitute an electronic cigarette.

The proposed invention also provides an embodiment of a method intended for controlling an aerosol generation device comprising a porous body storing liquid substance until vaporized and a heater arranged, when supplied with electrical power, for heating this porous body to vaporize liquid substance for generating an aerosol, during a vaping session comprising successive inhalation phases during which a user inhales this generated aerosol.

This method is characterized in that it comprises a step in which the heater is supplied during the vaping session with a maximal electrical power that decreases between a first inhalation phase and a last inhalation phase.

The proposed invention also provides an embodiment of a computer program product comprising a set of instructions arranged, when executed by processing means, to perform the method presented above for controlling an aerosol generation device comprising a porous body storing liquid substance until vaporized and a heater arranged, when supplied with electrical power, for heating this porous body to vaporize the liquid substance for generating an aerosol, during a vaping session comprising successive inhalation phases during which a user inhales this generated aerosol.

### Brief description of the figures

The invention and its advantages will be better understood upon reading the following detailed description, which is given solely by way of non-limiting examples and which is made with reference to the appended drawings, in which:
- the figure 1 (FIG.1) schematically and functionally illustrates an example of embodiment of an aerosol generation device comprising a control device according to the invention, and
- the figure 2 (FIG.2) schematically illustrates in a diagram an example of temporal evolution of the supplied electrical power (curve c1 in the lower part with reference to the right y-axis), and a corresponding temporal evolution of the porous body temperature (curve c2 in the upper part with reference to the left y-axis).

### Detailed description of embodiments

The invention aims, notably, at offering a control device 1 intended for equipping an aerosol generation device 2 comprising a porous body 3 storing liquid substance in order to control precisely the electrical power supplied to the heater 4 during a vaping session.

In the following description it will be considered that the aerosol generation device 2 is (or constitutes) an electronic cigarette (or e-cigarette or else personal vaporizer). But an aerosol generation device according to the invention could be of another type, as soon as it allows the generation of an aerosol by heating a liquid substance stored in a porous body.

It is recalled that a "liquid substance" may comprise one or more active components such as nicotine, polyol or caffeine and/or a flavoring.

It is also recalled that the term "aerosol" may include a suspension of substance as one or more of solid particles, liquid droplets and gas, and that such a suspension may be in a gas including air.

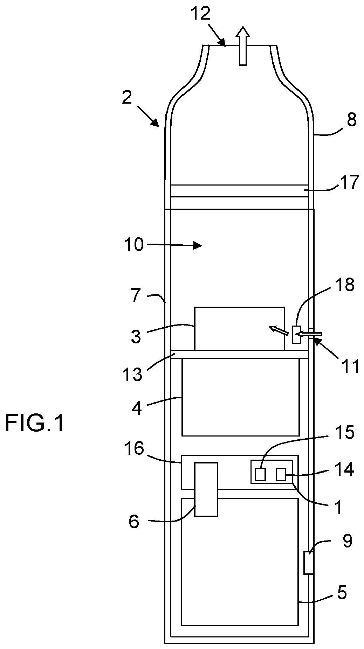

As illustrated in figure 1 an aerosol generation device 2 according to the invention comprises at least a battery (or power source) 5, a heater 4, a porous body 3, and a control device (or controller or else processing means (or unit)) 1, and also preferably a user interface 6.

For instance, and as illustrated in the non-limiting example of figure 1, the battery 5, control device 1, heater 4, porous body 3, and possible user interface 6 may belong to a first body 7 to which a second body 8 is coupled (for instance by magnets, clipping, or screwing by means of two corresponding threaded portions, as non-limiting examples). But in a variant the aerosol generation device 2 could comprise a single body comprising all its elements (or components).

The battery 5 is arranged for storing electrical energy that is necessary to the working of the heater 4, control device 1 and user interface 6. For instance, the battery 5 may be rechargeable, and in this case, and as illustrated in the non-limiting example of figure 1, the first body 7 may comprise an electrical connector 9 to which a charger cable may be connected during a charging session of the battery 5. Such a charger cable may be coupled to an (AC) adapter or to a wall socket. The charger cable and/or the (AC) adapter may belong to the aerosol generation device 2.

The possible user interface 6 is coupled to the control device 1, at least arranged for allowing the user to control the control device 1 and at least for switching on and switching off the aerosol generation device 2. So, it may comprise a button and/or a digital display (or screen) arranged for displaying information relative to a current vaping session or a possible current charging session.

In the non-limiting example of figure 1 the first body 7 comprises an open housing 10 in which the porous body 3 can be manually installed by the user after decoupling of the second body 8 from the first body 7. So, when the first 7 and second 8 bodies are coupled one to the other, the open housing 10 constitutes a vaporization chamber that communicates with the second body 8. But in the above mentioned variant (where the aerosol generation device 2 comprises a single body comprising all its elements), the porous body 3 can be manually installed by the user through a sliding tray of this single body, for instance.

Moreover, the first body 7 comprises at least one air inlet 11 allowing the air coming from outside to reach the vaporization chamber 10 when the user sucks through an outlet 12 of the second body 8. This outlet 12 is defined in a mouthpiece which may be integral with the second body 8 or an interchangeable part of the second body 8.

In the non-limiting illustrated example each air inlet 11 communicates directly with the vaporization chamber 10. But this is not mandatory.

For instance, and as illustrated in the non-limiting example of figure 1, the second body 8 may comprise a filter 17 upstream the mouthpiece. This filter 17 is crossed by the generated aerosol before reaching the outlet 12.

The porous body 3 may be a tablet, cylinder or disk, and may be a piece of porous ceramic, sintered glass, alumina or sintered metal soaked, injected or infused with liquid substance in order that the latter is held in place by capillary action (and also possibly stored in a cavity).

For instance, and as illustrated in the non-limiting example of figure 1, the heater 4 may be in contact with, or very near to, the wall 13 on which the porous body 3 is installed. Also for instance, the heater 4 may comprise a resistive wire coated in a ceramic. But any type of electrically activated heating element allowing heating of the porous body 3 by conduction, convection and/or radiation may be used.

As will be explained below, when the heater 4 is supplied with a sufficient electrical power under control of the control device 1, the porous body 3 reaches a vaporization temperature which allows vaporization of a part of its stored liquid substance and therefore the generation of an aerosol (or mist) by mixing with the air coming from outside through the air inlet 11.

The control device 1, according to the invention, comprises at least a processor 14 and a memory 15 arranged for performing operations consisting of controlling precisely supply of the heater 4 with electrical power during each vaping session comprising successive inhalation phases during which a user inhales the aerosol generated in the vaporization chamber 10.

More precisely the processor 14 and memory 15 are arranged for performing operations consisting of controlling supply of the heater 4, during a vaping session, with a maximal electrical power that decreases between the first and last inhalation phases.

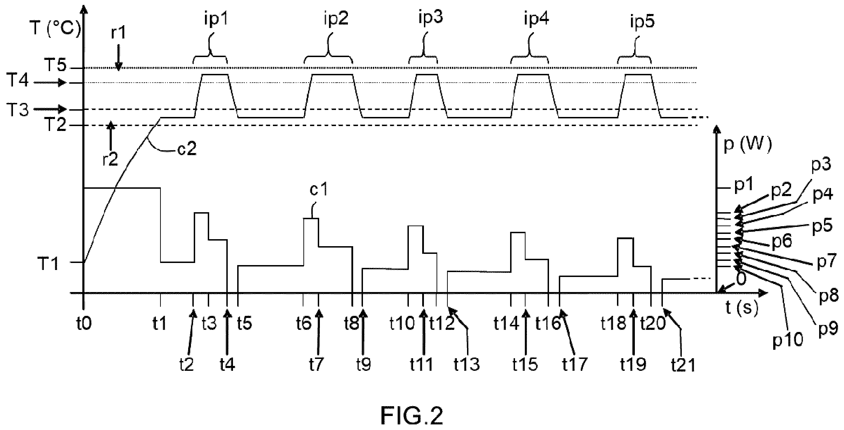

In other words, and as illustrated in the non-limiting example of figure 2, during the first inhalation phase (or puff) ip1 of a vaping session the heater 4 is supplied (at least temporarily) with a first maximal electrical power p2, then during the second inhalation phase (or puff) ip2 of this vaping session the heater 4 is supplied (at least temporarily) with a second maximal electrical power p3 smaller than p2, then during the third inhalation phase (or puff) ip3 of this vaping session the heater 4 is supplied (at least temporarily) with a third maximal electrical power p4 smaller than p3, then during the fourth inhalation phase (or puff) ip4 of this vaping session the heater 4 is supplied (at least temporarily) with a fourth maximal electrical power p5 smaller than p4, then during the fifth inhalation phase (or puff) ip5 of this vaping session the heater 4 is supplied (at least temporarily) with a fifth maximal electrical power p6 smaller than p5, and so on till the last inhalation phase (or puff) of this vaping session.

So, during a vaping session the decrease of the maximal electrical power supplying the heater 4 compensates (at least partly) for the consequence of the decrease of the porous body heat capacity (induced by the decrease of the quantity of stored liquid substance). Therefore, the temperature of the porous body 3 does not increase significantly from an inhalation phase to the following one, and then there is no more vaporization between two inhalation phases or perceptible temperature variations and compositions of the aerosol. Indeed there is no increase or inconsistency in vapor output between successive puffs, no alteration of the taste or taste inconsistency, no inconsistency of the constituents, and no production of higher levels of undesirable constituents.

The control device 1 may be informed of the beginning and end of each inhalation phase (or puff) ipj by a signal sent by an inhalation sensor 18 positioned in the air flow path (between the air inlet 11 and the outlet 12) in the aerosol generation device 2. This inhalation sensor 18 may be a flow or pressure sensor or a microphone, for instance. In the non-limiting example illustrated in figure 1 the inhalation sensor 18 is located inside the first body 7, but in a variant it could be located inside the second body 8 (upstream the outlet 12) or located on the printed circuit board 16.

For instance, the processor 14 may be a digital signal processor, or an application specific integrated circuit (ASIC), or else a field programmable gate array (FPGA). More generally, the processor 14 may comprise integrated (or printed) circuits, or several integrated (or printed) circuits connected therebetween through wired or wireless connections. The term "integrated (or printed) circuits" refers here to any type of device capable of carrying out at least one electric or electronic operation.

Also for instance, the memory 15 may be a random access memory (RAM). But it may be any type of device arranged for storing program instructions for the processor 14.

So the control device 1 is a combination of hardware and software.

Also for instance, and as illustrated in the non-limiting example of figure 1, the control device 1 may be fixed onto a printed circuit board 16 (here housed in the first body 7). The possible user interface 6 may be also fixed onto this printed circuit board 16, as illustrated.

In an example of embodiment the processor 14 and memory 15 may be arranged for performing operations consisting of controlling supply of the heater 4 with an approximately constant maximal electrical power and then an approximately constant first intermediate electrical power, smaller than this approximately constant maximal electrical power, during respectively first and second successive parts of each inhalation phase. The approximately constant maximal electrical power decreases from an inhalation phase to the following one, between the first and last inhalation phases.

The term "approximately constant" is used in all the description because the electrical power may be supplied by a thermocouple to maintain a constant temperature (or a temperature between lower and upper values), or may be pulse width modulated to approximate a constant power output, for instance.

The result of this embodiment is illustrated in figure 2 between t2 and t4 for the first puff ip1, between t6 and t8 for the second puff ip2, between t10 and t12 for the third puff ip3, between t14 and t16 for the fourth puff ip4, and between t18 and t20 for the fifth puff ip5.

The respective values of the approximately constant maximal electrical power and approximately constant first intermediate electrical power in each puff ipj (here j = 1 to 5) are chosen in order that the porous body temperature T (curve c2 in figure 2) remains in a vaporization range r1 (between T4 and T5). For instance, T4 may be comprised between 100°C and 300°C, and T5 may be comprised between 100°C and 300°C.

For instance, the processor 14 and memory 15 may be arranged for performing operations consisting of controlling supply of the heater 4 with an approximately constant first intermediate electrical power that decreases from an inhalation phase to the following one, between the first and last inhalation phases. The result of this embodiment is illustrated in figure 2. The first puff ip1 starts at t2 with the supply of a first approximately constant maximal electrical power p2 until t3, and goes on from t3 to t4 with the supply of a first approximately constant first intermediate electrical power p6 (< p2). The second puff ip2 starts at t6 with the supply of a second approximately constant maximal electrical power p3 (< p2) until t7, and goes on from t7 to t8 with the supply of a second approximately constant first intermediate electrical power p7 (< p6). The third puff ip3 starts at t10 with the supply of a third approximately constant first maximal electrical power p4 (< p3) until t11, and goes on from t11 to t12 with the supply of a third approximately constant first intermediate electrical power p8 (< p7). The fourth puff ip4 starts at t14 with the supply of a fourth approximately constant maximal electrical power p5 (< p4) until t15, and goes on from t15 to t16 with the supply of a fourth approximately constant first intermediate electrical power p9 (< p8). The fifth puff ip5 starts at t18 with the supply of a fifth approximately constant maximal electrical power p6 (< p5) until t19, and goes on from t19 to t20 with the supply of a fifth approximately constant first intermediate electrical power p10 (< p9).

In the non-limiting example of figure 2, temperatures T4 and T5 are constant. But in a variant they could have a slight increase between successive puffs to promote the vaporization of liquid substance due to the lower quantity of liquid substance contained within the pores potentially reducing the amount of generated aerosol. Here, allowing a slight increase in T4 and T5 temperatures could help promote aerosol generation (negating the increasing difficulty of aerosol spacing the porous body). For instance, T4 and T5 temperatures may both increase from 0°C to 2°C per puff. So, over twenty puffs the total temperature increase of T4 and T5 would likely be no more than 20°C, which means an increase of 1°C/puff.

In another example of embodiment, which may be combined with the preceding one, the processor 14 and memory 15 may be arranged for performing operations consisting of controlling supply of the heater 4 with an approximately constant second intermediate electrical power after the end of an inhalation phase ipj and before the beginning of a following inhalation phase ipj+1. This embodiment is intended for maintaining the porous body temperature T at a value that is greater than the ambient temperature T1 and not too far from the vaporization range r1 between two successive puffs ipj and ipj+1.

The value of the approximately constant second intermediate electrical power before each puff ipj (here j = 1 to 5) is chosen in order that the porous body temperature T (curve c2 in figure 2) remains always in an idle range r2 (between T2 and T3, with T2 > T1 (ambient temperature) and T3 < T4) between two successive puffs ipj and ipj+1.
For instance, T2 may be comprised between 100°C and 200°C, and T3 may be comprised between 100°C and 200°C.

To guarantee that the porous body temperature T (curve c2 in figure 2) will remain in an idle range r2 (between T2 and T3) between two successive puffs ipj and ipj+1, the processor 14 and memory 15 may be arranged for performing operations consisting of controlling supply of the heater 4, before an inhalation phase, with an approximately constant second intermediate electrical power that is lower than the approximately constant first intermediate electrical power of the inhalation phase. So, if the value of the first intermediate electrical power decreases from a puff ipj to the following one ipj+1, then the value of the second intermediate electrical power preferably decreases between the idle phases occurring before the first puff ip1 (between t1 and t2) and the last idle phase occurring before the last puff ip5 (here between t17 and t18), as illustrated in figure 2.

It should be noticed than in order to decrease the electrical power consumption, the value of the electrical power supplying the heater 4 may be decreased to a low value at the beginning of each idle phase occurring between two successive puffs, and before becoming the approximately constant second intermediate electrical power. This is illustrated in figure 2 between t4 and t5 after the first puff ip1, between t8 and t9 after the second puff ip2, between t12 and t13 after the third puff ip3, between t16 and t17 after the fourth puff ip4, and between t20 and t21 after the fifth puff ip5. For instance, the low value may be equal to zero as illustrated, but this is not mandatory.

In another example of embodiment, which may be combined with any one of the preceding ones, the processor 14 and memory 15 may be arranged for performing operations consisting of controlling supply of the heater 4 with an approximately constant pre-heating electrical power p1 during a pre-heating phase occurring before the first inhalation phase ip1 (here between to and t1, as illustrated in figure 2).

For instance, the processor 14 and memory 15 may be arranged for performing operations consisting of controlling supply of the heater 4 with an approximately constant pre-heating electrical power p1 that is greater than the maximal electrical power p2 supplied during the first inhalation phase ip1. This is illustrated in figure 2. This high pre-heating electrical power p1 is intended for obtaining a high porous body temperature T5, greater than T4 (upper boundary of the vaporization range r1). For instance, this high porous body temperature T5 may be comprised between 100°C and 300°C.

In another example of embodiment, which may be combined with any one of the preceding ones, the processor 14 and memory 15 may be arranged for performing operations consisting of controlling an end of electrical power supply of the heater 4 when an event occurs. This event may be a termination of the vaping session by the user (triggered by means of the user interface 6), or an elapsed time having reached a first limit since the last inhalation phase, or else a duration of the vaping session exceeding a second limit. For instance, the first limit may be comprised between 2 minutes and 5 minutes. Also for instance, the second limit may be comprised between 5 minutes and 15 minutes.

The control device 1 may also comprise, in addition with its processor 14 and memory 15, an input interface, notably for receiving any information provided by the user interface 6 and concerning the processor 14. The control device 1 may also comprise an output interface for delivering messages and instructions at least for the electronic component(s) (such as switch(es)) supplying the electrical power (stored in the battery 5) to the heater 4.

The invention can be also considered as a method intended for controlling the aerosol generation device 2 during a vaping session. This method comprises a step in which the heater 4 is supplied with a maximal electrical power that decreases between the first and last inhalation phases of each vaping session.

The invention can be also considered as a computer program product comprising a set of instructions arranged, when executed by processing means (such as the processor 14), to perform the method presented in the preceding paragraph for controlling the electrical power that is supplied to the heater 4 of the aerosol generation device 2 during each vaping session.

The invention offers some advantages, amongst which an improvement of the user experience of the aerosol generation device 2 and of the porous body 3 by a reduction of the pre-heat time, an improvement of responsiveness (from inhalation or activation to vapor generation), and a consistency throughout the vaping session (i.e. from the first puff to the last puff where all liquid substance has been vaporized).

It should be appreciated by those skilled in the art that some block diagrams of figure 1 herein represent conceptual views of illustrative circuitry embodying the principles of the invention.

The description and drawings merely illustrate the principles of the invention. It will thus be appreciated that those skilled in the art will be able to devise various arrangements that, although not explicitly described or shown herein, embody the principles of the invention and are included within its spirit and scope. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

## Claims

1. Control device (1) for an aerosol generation device (2) comprising a porous body (3) storing liquid substance until vaporized and a heater (4) arranged, when supplied with electrical power, for heating said porous body (3) to vaporize said liquid substance for generating an aerosol, said control device (1) comprising at least a processor (14) and a memory (15) arranged for performing operations consisting of controlling supply of said heater (4) with electrical power during a vaping session comprising successive inhalation phases during which a user inhales said generated aerosol,
wherein said processor (14) and memory (15) are arranged for performing operations consisting of controlling supply of said heater (4), during said vaping session, with a maximal electrical power that decreases between a first inhalation phase and a last inhalation phase.

2. Control device according to claim 1, wherein said processor (14) and memory (15) are arranged for performing operations consisting of controlling supply of said heater (4) with an approximately constant maximal electrical power and then an approximately constant first intermediate electrical power, smaller than this approximately constant maximal electrical power, during respectively first and second successive parts of each inhalation phase, said approximately constant maximal electrical power decreasing between said first and last inhalation phases.

3. Control device according to claim 2, wherein said processor (14) and memory (15) are arranged for performing operations consisting of controlling supply of said heater (4) with an approximately constant first intermediate electrical power that decreases between said first and last inhalation phases.

4. Control device according to any one of claims 1 to 3, wherein said processor (14) and memory (15) are arranged for performing operations consisting of controlling supply of said heater (4) with an approximately constant second intermediate electrical power after an end of an inhalation phase and before a beginning of a following inhalation phase.

5. Control device according to the combination of claims 3 and 4, wherein said processor (14) and memory (15) are arranged for performing operations consisting of controlling supply of said heater (4), before an inhalation phase, with an approximately constant second intermediate electrical power that is lower than the approximately constant first intermediate electrical power of said inhalation phase.

6. Control device according to any one of claims 1 to 5, wherein said processor (14) and memory (15) are arranged for performing operations consisting of controlling supply of said heater (4) with an approximately constant pre-heating electrical power during a pre-heating phase occurring before said first inhalation phase.

7. Control device according to any one of claims 1 to 6, wherein said processor (14) and memory (15) are arranged for performing operations consisting of controlling supply of said heater (4) with an approximately constant pre-heating electrical power that is greater than the maximal electrical power supplied during said first inhalation phase.

8. Control device according to any one of claims 1 to 7, wherein said processor (14) and memory (15) are arranged for performing operations consisting of controlling an end of electrical power supply of said heater (4) when occurs an event chosen from a group comprising a termination of said vaping session by said user, an elapsed time having reached a first limit since a last inhalation phase, and a duration of said vaping session exceeding a second limit.

9. Aerosol generation device (2) comprising :
- a battery (5) storing electrical energy,
- a porous body (3) storing liquid substance until vaporized, and
- a heater (4) arranged, when supplied with electrical power, for heating said porous body (3) to vaporize said liquid substance for generating an aerosol during a vaping session comprising inhalation phases during which a user inhales said generated aerosol,
wherein it further comprises a control device (1) according to one of the preceding claims, electrically coupled to said battery (5).

10. Aerosol generation device according to claim 9, wherein said battery (5) is rechargeable.

11. Aerosol generation device according to claim 9 or 10, wherein it constitutes an electronic cigarette.

12. Method for controlling an aerosol generation device (2) comprising a porous body (3) storing liquid substance until vaporized and a heater (4) arranged, when supplied with electrical power, for heating said porous body (3) to vaporize said liquid substance for generating an aerosol, during a vaping session comprising successive inhalation phases during which a user inhales said generated aerosol, wherein it comprises a step in which said heater (4) is supplied during said vaping session with a maximal electrical power that decreases between a first inhalation phase and a last inhalation phase.

13. Computer program product comprising a set of instructions arranged, when executed by processing means, to perform the method according to claim 12 for controlling an aerosol generation device (2) comprising a porous body (3) storing liquid substance until vaporized and a heater (4) arranged, when supplied with electrical power, for heating said porous body (3) to vaporize said liquid substance for generating an aerosol, during a vaping session comprising successive inhalation phases during which a user inhales said generated aerosol.
